(19) Europäisches Patentamt
European Patent Office
Office européen des brevets

(11) Publication number: **0 023 370**
**B1**

(12) **EUROPEAN PATENT SPECIFICATION**

(45) Date of publication of patent specification: **13.07.83**

(51) Int. Cl.³: **C 07 C 11/18**, C 07 C 7/00

(21) Application number: **80200652.8**

(22) Date of filing: **04.07.80**

(54) **Process for the recovery of isoprene, and isoprene so recovered.**

(30) Priority: **18.07.79 GB 7925028**

(43) Date of publication of application:
**04.02.81 Bulletin 81/5**

(45) Publication of the grant of the patent:
**13.07.83 Bulletin 83/28**

(84) Designated Contracting States:
**BE DE FR GB IT NL**

(56) References cited:
**GB - A - 1 243 959**
**US - A - 2 360 859**
**US - A - 3 520 947**
**US - A - 3 851 010**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Douwes, Henricus Simeon Antonius**
**Carel van Bylandtlaan 30**
**NL-2596 HR The Hague (NL)**
Inventor: **Lap, Johannes Epicarus Gerardus Maria**
**Carel van Bylandtlaan 30**
**NL-2596 HR The Hague (NL)**
Inventor: **Roukema, Kornelis**
**Badhuisweg 3**
**NL-1031 CM Amsterdam (NL)**
Inventor: **Jongen, François Chretien Joseph**
**8 Wood Lane**
**Goostrey Cheshire (GB)**

(74) Representative: **Puister, Antonius Tonnis, Mr. et al,**
**P.O. Box 302**
**NL-2501 CH The Hague (NL)**

**EP 0 023 370 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the recovery of isoprene, and isoprene so recovered

The present invention is concerned with a process for recovering isoprene from a $C_5$ hydrocarbon fraction and with the isoprene so recovered.

Isoprene is a well-known product and is used for many purposes, such as the manufacture of homo- or copolymers of isoprene. Isoprene may be prepared by various techniques, such as by the pyrolysis of mineral oil fractions.

In cracking mineral oil fractions, such as naphtha, gas oil and paraffin wax for the preparation of ethylene, considerable quantities of olefinically unsaturated compounds having more than 2 carbon atoms are formed. Considerable quantities of isoprene occur in the hydrocarbon fraction having predominantly five carbon atoms. This $C_5$ hydrocarbon fraction may be separated from the other fractions by techniques, such as distillation.

The $C_5$ hydrocarbon fraction is a complex mixture of pentanes, i.e. isopentane and normal pentane, pentenes, pentadienes, including isoprene and possibly cyclopentadiene and pentynes. Various processes are known for recovering isoprene from this fraction. Such processes usually comprise a combination of one or more fractional distillation steps and one or more solvent extraction steps, such as liquid-liquid extraction and/or extractive distillation steps. Solvent extraction is necessary because the $C_5$ hydrocarbon fraction contains components, e.g. normal-pentane, which are very difficult to separate from isoprene using only fractional distillation techniques. Such processes may also include a thermal dimerization step to convert any cyclopentadiene to dicyclopentadiene which may be removed as the residue of a fractional distillation process.

Various extraction solvents are known from the literature. One class of solvent, particularly suitable for use in liquid-liquid extraction techniques, are the sulfolanes which are disclosed in U.S. 2,360,859 as being useful for separation of mixtures of similar organic compounds having different degrees of saturation, e.g. pentanes, pentenes and isoprene.

The Applicants have now found a novel process for the recovery of isoprene in which use is made of a sulfolane in a liquid-liquid extraction step.

According to the present invention a process for recovering isoprene from a $C_5$ hydrocarbon fraction comprising a mixture of pentanes, i.e. isopentane and normal-pentane, pentenes, pentadienes, including isoprene, and pentynes and which possibly also comprises small amounts of various hydrocarbons having carbon numbers below $C_5$ and/or above $C_5$ comprises subjecting the hydrocarbon fraction to the following stages which may be carried out in any order with the exception that the heavy hydrocarbon removal stage should not precede the liquid-liquid extraction stage:

(i) a light hydrocarbon removal stage to remove, by means of fractional distillation, those hydrocarbons, present in the hydrocarbon fraction, which boil at a temperature below the boiling point of isoprene;

(ii) a liquid-liquid extraction stage using in a first step a sulfolane as the extractant to form a first raffinate phase rich in normal pentane and pentenes and a sulfolane extract phase rich in isoprene, subjecting the sulfolane extract phase to a second liquid-liquid extraction step using a counter-solvent, which is a $C_6$ or higher alkane, as the extractant to form a second raffinate phase comprising mainly the sulfolane and a counter-solvent extract phase rich in isoprene and fractionally distilling the counter-solvent extract phase to remove the counter-solvent therefrom;

(iii) a heavy hydrocarbon removal stage to remove, by means of fractional distillation, those hydrocarbons, present in the hydrocarbon fraction, which boil at a temperature above the boiling point of isoprene.

The above novel process results in an isoprene recovery process which is economically attractive especially in so far as the utilization of heat is concerned; for example, it is not necessary to apply to, or withdraw from, the isoprene-containing fractions substantial amounts of heat between the stages. A further advantage of the process is that any dicyclopentadiene which is present in the feedstock is substantially completely removed via the first raffinate phase. The present invention is particularly suitable for preparing isoprene concentrates comprising from 75 to 99.9% w of isoprene.

As stated above the stages of the isoprene recovery process may be carried out in any order with the exception that the heavy hydrocarbon removal stage should not precede the liquid-liquid extraction stage. Thus, the sequence of stages may be (a) the light hydrocarbon removal stage followed by the two-step liquid-liquid extraction stage followed by the heavy hydrocarbon removal stage or (b) the liquid-liquid extraction stage followed by the light hydrocarbon removal stage followed by the heavy hydrocarbon removal stage or (c) the liquid-liquid extraction stage followed by the heavy hydrocarbon removal stage followed by the light hydrocarbon removal stage. However, the above sequence (a) is preferred.

According to this preferred aspect of the present invention a process for recovering isoprene from a $C_5$ hydrocarbon fraction, comprising a mixture of pentanes, i.e.

isopentane and normal-pentane, pentenes, pentadienes, including isoprene, and pentynes and which possibly also comprises small amounts of various hydrocarbons having carbon numbers below $C_5$ and/or above $C_5$, comprises:

(i) fractional distillation of the hydrocarbon fraction to obtain a first distillate fraction, comprising mainly hydrocarbons which boil at a temperature below the boiling point of isoprene, and a first residual fraction comprising the remainder of the hydrocarbon fraction;

(ii) extraction of isoprene and hydrocarbons which boil above the boiling point of isoprene from the first residual fraction by a series of steps comprising:

(a) subjecting the first residual fraction to liquid-liquid extraction by contacting the residual fraction with a sulfolane to obtain a first raffinate phase rich in normal-pentane and pentenes and a first extract phase comprising the sulfolane and the remainder of the residue;

(b) subjecting the first extract phase to liquid-liquid extraction with the counter-solvent comprising a $C_6$ or higher alkane to obtain a second raffinate phase comprising mainly the sulfolane and a second extract phase comprising mainly the counter-solvent, isoprene and other hydrocarbons which boil at a temperature above the boiling point of isoprene, and

(c) fractionally distilling the second extract phase to obtain a second residual fraction comprising mainly the counter-solvent and a second distillate fraction comprising mainly isoprene and hydrocarbons which boil at a temperature above the boiling point of isoprene, and

(iii) fractional distillation of the second distillate to obtain a third residual fraction comprising mainly hydrocarbons which boil at a temperature above the boiling point of isoprene and a third distillate fraction comprising mainly isoprene.

As stated above the $C_5$ hydrocarbon fraction may contain cyclopentadiene which may be converted to dicyclopentadiene by thermal dimerization ("heat-soaking"). The dimerization of cyclopentadiene may take place at various stages of the recovery process, e.g. before or after the solvent-extraction stage or before or after the various distillation stages; however, in a preferred embodiment of the present invention the cyclopentadiene is dimerized before the $C_5$ hydrocarbon fraction is subjected to any one of the above stages. The dicyclopentadiene thus formed may be removed by any conventional technique such as the residue of a fractional distillation process; however, an advantage of the present invention is that substantially all of the dicyclopentadiene is removed via the first raffinate phase.

It may be desirable to operate the liquid-liquid extraction step with a sulfolane in such a way that not all of the normal-pentane is removed in the raffinate phase in which case any remaining normal-pentane in the sulfolane extract phase may be removed therefrom by stripping before the liquid-liquid extraction step with the counter-solvent. It may also be desirable to subject the isoprene fraction to a purification treatment, e.g. to remove any residual acetylenes therefrom; such purification treatments include sodium or selective hydrogenation treatments.

The sulfolane for use in the present invention is preferably sulfolane itself which has the formula:

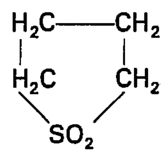

although derivatives may also be used, such as those compounds wherein one or more of the hydrogen atoms is replaced by an organic radical which may contain a polar grouping and more specifically may contain oxygen, nitrogen, sulphur and/or halide atoms. In hydrocarbon-substituted sulfolanes the hydrocarbon radicals are preferably alkyl radicals. A suitable hydrocarbon-substituted sulfolane is methyl sulfolane. Sulfolane derivatives containing oxygen include hydroxy sulfolanes, sulpholanyl-ethers and -esters; sulfolane derivatives containing nitrogen include sulpholanyl-amines, -nitriles and nitro-sulfolanes; sulfolane derivatives containing sulphur include sulpholanyl sulphides, -sulphoxides and -sulphones. Other sulfolane derivatives may contain halide radicals, inorganic esters or mixed radicals of those above mentioned, such as acid amides, halohydrins, sulphonamides, etc. Derivatives may be made by condensing a conjugated diolefin with sulphur dioxide, and then subjecting the resultant product to hydrogenation, alkylation, hydration, amination, chlorination, nitration and/or other substitution or addition reactions.

The counter-solvent for use in the present invention is a $C_6$ or higher alkane. Such alkanes, which may be linear, branched or cyclic, are preferably $C_6$ to $C_8$ alkanes.

The invention is illustrated by reference to the accompanying drawing which is a schematic flow plan. It will be understood that various pieces of equipment which are not essential for an understanding of the present invention, e.g. storage vessels, accumulators, pumps and heaters are not shown in the drawing.

A feedstock 1 comprising the $C_5$ hydro-

carbon fraction is fed to a fractional distillation column $D_1$. Suitably the column is operated at superatmospheric pressure, e.g. from 1.1 to 10.0 bars absolute and elevated temperatures, e.g. from 50 to 150°C. A first distillate fraction, comprising mainly hydrocarbons which boil at a temperature below the boiling point of isoprene is withdrawn as stream 2 from the top of the column and a first residual fraction comprising the remainder of the hydrocarbon fraction is withdrawn as stream 3 from the bottom of the column $D_1$ and fed to the extraction zone $E_1$.

Suitably, the stream 3 is fed to the lower section of the zone. A stream 5 of sulfolane is fed to the top of this zone and flows in counter-current to the first residual fraction. The extraction zone is suitably operated at superatmospheric pressure, e.g. from 1.1 to 10 bars absolute and from 50 to 150°C. An important advantage of the present invention is that the first residual fraction may be fed to the extraction zone at substantially the same temperature as it leaves the distillation zone $D_1$, i.e. it is possible to achieve efficient extraction in the extraction zone $E_1$ without first lowering its temperature. A first raffinate phase, comprising mainly normal-pentane and pentenes, is withdrawn as stream 4 from the top of the zone. This first raffinate phase in general is composed of those $C_5$ hydrocarbons, e.g. normal-pentane which are difficult to separate easily from isoprene by fractional distillation techniques and also any dicyclopentadiene present in the feedstock. A first extract phase, comprising sulfolane and the remainder of the first residual fraction, is withdrawn as stream 6 from the bottom of the zone and may be fed to a stripping zone S or directly to the second extraction zone $E_2$ depending on whether or not the extract phase comprises a significant amount of normal-pentane. If it does contain a significant amount of normal-pentane then the extract phase is fed to the stripping zone S; if it does not, then it is fed directly to the second extraction zone $E_2$.

The stripping zone S suitably comprises a stripping column operating at a lower pressure than the extraction zone and suitably at a temperature of from 50 to 150°C. From the top of the stripper is withdrawn a stream 7 which is rich in normal-pentane. In order to ensure substantially complete removal of normal-pentane from the extract phase it may be necessary to remove simultaneously some pentenes and pentadienes therefrom in which case it is advantageous to recycle stream 7 to the lower section of extraction zone $E_1$. The bottom stream 8 from the stripper 8, comprising the remainder of the first extract phase, is fed to the second extraction zone $E_2$.

Suitably, the extract phase is fed to the upper section of the extraction zone $E_2$. The $C_6$ or higher alkane, which acts as the counter-solvent, is fed as stream 10 to the lower section of the zone and flows in counter-current to the first extract phase and re-extracts therefrom substantially all of the $C_5$ hydrocarbons present therein. The second extraction zone $E_2$ is suitably operated at superatmospheric pressure, e.g. from 1.1 to 10 bars absolute and from 50 to 150°C. A second raffinate phase is withdrawn from the lower section of the zone. This phase comprises mainly sulfolane which is advantageously recycled, together with make-up sulfolane, if necessary, added as stream 14, to the first extraction zone $E_1$ as stream 5. A second extract phase is formed, comprising mainly the counter-solvent, isoprene and $C_5$ hydrocarbons which boil above the boiling point of isoprene, and is withdrawn as stream 9 from the top section of the zone and fed to the second fractional distillation column $D_2$.

The distillation column $D_2$ is suitably operated at a bottom temperature of from 80 to 120°C and a pressure of from 1.2 to 3 bars absolute. Suitably the second extract phase, if necessary, is heated to a temperature of from 80 to 120°C before it is fed to the column. A second residual fraction is obtained which is withdrawn from the bottom of the column. This second residual fraction comprises mainly the counter-solvent and is advantageously recycled, together with make-up counter-solvent, if necessary, added as stream 15, to the second extraction zone $E_2$ as stream 10. A second distillate fraction, comprising mainly isoprene and $C_5$ hydrocarbons which boil above the boiling point of isoprene, is withdrawn as stream 11 from the top of the distillation column $D_2$ and fed to the third fractional distillation column $D_3$.

The distillation column $D_3$ is suitably operated at a temperature of from 50 to 150°C and a pressure of from 1.2 to 3 bars absolute. A third residual fraction is obtained, comprising mainly $C_5$ hydrocarbons boiling above the boiling point of isoprene, which is withdrawn as stream 13 from the bottom of the column. A third distillate fraction is obtained comprising mainly isoprene which is withdrawn as stream 12 from the top of the column.

The invention will now be illustrated with reference to the following Examples.

Example 1

A $C_5$ hydrocarbon fraction having the following composition was fed at a rate of 30 kg/h as stream 1 to a first distillation column $D_1$ operating at a bottom temperature of 70°C and a top temperature of 50°C and a top pressure of 2.0 bars absolute.

| | Stream 1 (%w) |
|---|---|
| Isoprene | 23.6 |
| Cyclopentadiene | 7.3 |
| Other pentadienes | 19.6 |
| n-Pentane | 5.9 |
| iso-Pentane | 0.7 |
| Pentenes | 37.6 |
| Others | 5.3 |

From the top of the column was withdrawn as stream 2 a distillate fraction and from the bottom of the column was withdrawn as stream 3 a residual fraction. These streams had the following compositions:

| | Stream 2 (%w) | Stream 3 (%w) |
|---|---|---|
| Isoprene | 5.3 | 28.8 |
| Cyclopentadiene | 0.1 | 9.3 |
| Other pentadienes | 11.3 | 22.0 |
| n-Pentane | 0.4 | 7.5 |
| iso-Pentane | 3.1 | — |
| Pentenes | 75.3 | 26.8 |
| Others | 4.5 | 5.6 |

Stream 3 was fed at a rate of 23 kg/h to the lower section of the first extraction zone $E_1$. A stream 5 having the following composition was fed at a rate of 223 kg/h to the upper section of the zone. A further stream 7, having the composition given below, was fed at a rate of 21 kg/h to the lower section of the zone. The temperature of the zone was 70°C and the pressure was 4 bars absolute.

| | Stream 5 (%w) |
|---|---|
| Sulfolane | 97.0 |
| Heptane | 2.4 |
| Others | 0.6 |

From the top of the zone was withdrawn as stream 4 a first raffinate phase and from the bottom of the zone was withdrawn as stream 6 a first extract phase. These streams had the following compositions:

| | Stream 4 (%w) | Stream 6 (%w) |
|---|---|---|
| Isoprene | 1.6 | 5.4 |
| Cyclopentadiene | — | 1.3 |
| Other pentadienes | 1.0 | 3.6 |
| n-Pentane | 12.4 | 0.6 |
| Pentenes | 31.1 | 3.5 |
| Heptane | 41.5 | — |
| Sulfolane | 3.3 | 85.0 |
| Others | 9.1 | 0.6 |

Stream 6 was fed at a rate of 254 kg/h to the top section of the stripper zone S, consisting of a stripping column, operating at a bottom temperature of 70°C, a top temperature of 55°C and a pressure of 1.3 bars absolute, from the top of the stripper was withdrawn as stream 7 a normal-pentane fraction, which was recycled to the lower section of extraction column $E_1$, and from the bottom of the stripper was withdrawn as stream 8 the remainder of the first extract phase. These streams had the following compositions:

| | Stream 7 (%w) | Stream 8 (%w) |
|---|---|---|
| Isoprene | 35.3 | 2.8 |
| Cyclopentadiene | 5.7 | 0.9 |
| Other pentadienes | 19.1 | 2.2 |
| n-Pentane | 6.8 | 0.1 |
| Pentenes | 32.8 | 1.0 |
| Sulfolane | — | 92.5 |
| Others | 0.3 | 0.5 |

Stream 8 was fed at a rate of 233 kg/h to the upper section of the second extraction zone $E_2$. A further stream 10 having the following composition was fed at a rate of 105 kg/h to the lower section of the zone. The temperature of the zone was 70°C and the pressure was 5 bars absolute.

| | Stream 10 (%w) |
|---|---|
| n-Heptane | 95.1 |
| Others | 4.9 |

From the top of the zone was withdrawn as stream 9 a second extract phase having the following composition and from the bottom of the zone was withdrawn a second raffinate phase which together with make-up sulfolane (stream 14) is recycled to the first extraction zone as stream 5.

| | Stream 9 (%w) |
|---|---|
| Isoprene | 5.7 |
| Cyclopentadiene | 1.9 |
| Other pentadienes | 4.4 |
| n-Pentane | 0.1 |
| Pentenes | 2.0 |
| Heptane | 81.5 |
| Sulfolane | 2.3 |
| Others | 2.1 |

Stream 9 was heated to a temperature of 90°C and fed at a rate of 116 kg/h to the upper section of the second fractional distillation column $D_2$ operating at a bottom temperature of 130°C and a top temperature of 50°C and a pressure of 1.2 bars absolute. From the bottom of the column was withdrawn the second residual fraction which together with make-up n-heptane (stream 15) is recycled to the second extraction zone $E_2$ as stream 10. From the top of the column was withdrawn as stream 11 the second distillate fraction which had the following composition:

| | Stream 11 (%w) |
|---|---|
| Isoprene | 40.2 |
| Cyclopentadiene | 13.4 |
| Other pentadienes | 30.8 |
| n-Pentane | 0.8 |
| Pentenes | 13.9 |
| Others | 0.9 |

Stream 11 was fed at a rate of 16 kg/h to the middle section of a third fractional distillation column $D_3$, operating at a bottom temperature of 40°C and a pressure of 1.3 bars absolute. From the bottom of the column was withdrawn as stream 13 the third residual fraction and from the top of the column was withdrawn as stream 12 the third distillate fraction. These streams had the following compositions:

| | Stream 12 (%w) | Stream 13 (%w) |
|---|---|---|
| Isoprene | 85.4 | 2.2 |
| Cyclopentadiene | 0.2 | 24.5 |
| Other pentadienes | — | 56.6 |
| n-Pentane | 1.8 | — |
| Pentenes | 12.6 | 15.1 |
| Others | — | 1.6 |

Example 2

Example 1 was repeated using a feedstock containing a substantial amount of dicyclopentadiene (DCPD). The compositions (%w) of the streams are given in the following Table from which it can be seen that substantially all of the DCPD is removed in the first raffinate phase (stream 4).

| Components | Stream 1 | 2 | 3 | 4 | 5 | 6 | 7 | 8 | 9 | 10 | 11 | 12 | 13 |
|---|---|---|---|---|---|---|---|---|---|---|---|---|---|
| Isoprene | 19.5 | 5.3 | 22.7 | 1.0 | — | 4.3 | 35.3 | 2.2 | 3.9 | — | 40.2 | 85.4 | 2.2 |
| Cyclopentadiene | 6.0 | 0.1 | 7.4 | — | — | 1.0 | 5.7 | 0.7 | 1.3 | — | 13.4 | 0.2 | 24.5 |
| DCPD | 18.8 | — | 23.0 | 32.5 | 2.0 | 1.8 | — | 1.9 | 16.3 | 18.0 | — | — | — |
| Other pentadienes | 16.2 | 11.3 | 17.4 | 0.6 | — | 2.8 | 19.1 | 1.7 | 3.0 | — | 30.8 | — | 56.6 |
| n-Pentane | 4.9 | 0.4 | 5.9 | 7.7 | — | 0.5 | 6.8 | — | 0.1 | — | 0.8 | 1.8 | — |
| Iso-Pentane | 0.6 | 3.1 | — | — | — | — | — | — | — | — | — | — | — |
| Pentenes | 31.2 | 75.3 | 21.2 | 19.2 | — | 2.7 | 32.8 | 0.7 | 1.3 | — | 13.9 | 12.6 | 15.1 |
| Heptane | — | — | — | 33.4 | 2.4 | — | — | — | 71.9 | 79.6 | — | — | — |
| Sulfolane | — | — | — | 2.7 | 95.0 | 86.4 | — | 92.4 | 2.1 | 2.3 | — | — | — |
| Others | 2.8 | 4.5 | 2.4 | 2.9 | 0.6 | 0.5 | 0.3 | 0.4 | 0.1 | 0.1 | 0.9 | — | 1.6 |

**Claims**

1. A process for recovering isoprene from a $C_5$ hydrocarbon fraction comprising a mixture of pentanes, i.e. isopentane and normal-pentane, pentenes, pentadienes, including isoprene, and pentynes and which possibly also comprises small amounts of various hydrocarbons having carbon numbers below $C_5$ and/or above $C_5$, characterized in subjecting the hydrocarbon fraction to the following stages which may be carried out in any order with the exception that the heavy hydrocarbon removal stage should not precede the liquid-liquid extraction stage:

(i) a light hydrocarbon removal stage to remove, by means of fractional distillation, those hydrocarbons, present in the hydrocarbon fraction, which boil at a temperature below the boiling point of isoprene;
(ii) a liquid-liquid extraction stage using in a first step a sulfolane as the extractant to form a first raffinate phase rich in normal pentane and pentenes and a sulfolane extract phase rich in isoprene, subjecting the sulfolane extract phase to a second liquid-liquid extraction step using a counter-solvent, which is a $C_6$ or higher alkane, as the extractant to form a second raffinate phase comprising mainly the sulfolane and a counter-solvent extract phase rich in isoprene and fractionally distilling the counter-solvent extract phase to remove the counter-solvent therefrom;
(iii) a heavy hydrocarbon removal stage to remove, by means of fractional distillation, those hydrocarbons, present in the hydrocarbon fraction, which boil at a temperature above the boiling point of isoprene.

2. A process as claimed in claim 1, characterized in that the sequence of stages is the light hydrocarbon removal stage, followed by the two-step liquid-liquid extraction stage, followed by the heavy hydrocarbon removal stage.

3. A process as claimed in claim 1 or claim 2, characterized in:

(i) fractional distillation of the hydrocarbon fraction to obtain a first distillate fraction, comprising mainly hydrocarbons which boil at a temperature below the boiling point of isoprene, and a first residual fraction comprising the remainder of the hydrocarbon fraction;
(ii) extraction of isoprene and hydrocarbons which boil above the boiling point of isoprene from the first residual fraction by a series of steps comprising:
 (a) subjecting the first residual fraction to liquid-liquid extraction by contacting the residual fraction with a sulfolane to obtain a first raffinate phase rich in normal-pentane and pentenes and a first extract phase comprising the sulfolane and the remainder of the residue;
 (b) subjecting the first extract phase to liquid-liquid extraction with the counter-solvent comprising a $C_6$ or higher alkane to obtain a second raffinate phase comprising mainly the sulfolane and a second extract phase comprising mainly the counter-solvent, isoprene and other hydrocarbons which boil at a temperature above the boiling point of isoprene, and
 (c) fractionally distilling the second extract phase to obtain a second residual fraction comprising mainly the counter-solvent and a second distillate fraction comprising mainly isoprene and hydrocarbons which boil at a temperature above the boiling point of isoprene, and
(iii) fractional distillation of the second distillate to obtain a third residual fraction comprising mainly hydrocarbons which boil at a temperature above the boiling point of isoprene and a third distillate fraction comprising mainly isoprene.

4. A process as claimed in any one of the preceding claims, characterized in that any remaining normal-pentane present in the first extract phase is removed therefrom by stripping before the phase is subjected to liquid-liquid extraction with the counter-solvent.

5. A process as claimed in any one of the preceding claims, characterized in that the sulfolane used in the first step of the liquid-liquid extraction stage is sulfolane itself.

6. A process as claimed in any one of the preceding claims, characterized in that the counter-solvent used in the second step of the liquid-liquid extraction stage is a $C_6$ to $C_8$ alkane.

**Revendications**

1. Un procédé de récupération d'isoprène à partir d'une fraction d'hydrocarbures en $C_5$ comprenant un mélange de pentanes, c'est-à-dire d'isopentane et de pentane normal, de pentènes, de pentadiènes, y compris l'isoprène, et de pentynes et qui comprend éventuellement aussi de petites quantités de divers hydrocarbures ayant des nombres d'atomes de carbone de moins de 5 et/ou de plus de 5, caractérisé en ce qu'on soumet la fraction d'hydrocarbures aux stades suivants qui peuvent être effectués dans un ordre quelconque à ceci près que le stade d'élimination des hydrocarbures lourds ne doit pas précéder le stade d'extraction liquide-liquide;

(1) un stade d'élimination des hydrocarbures légers pour éliminer, par distillation fractionnée, les hydrocarbures, présents dans la fraction d'hydrocarbures, qui bouillent à une

température plus basse que le point d'ébullition de l'isoprène;

(2) un stade d'extraction liquide-liquide utilisant dans une première étape un sulfolane comme agent d'extraction afin de former une première phase de raffinat riche en pentane normal et en pentènes et une phase d'extrait au sulfolane riche en isoprène, après quoi on soumet la phase d'extrait au sulfolane à une seconde étape d'extraction liquide-liquide en utilisant un contre-solvant, qui est un alcane en $C_6$ ou supérieur, comme agent d'extraction, afin de former une seconde phase de raffinat comprenant principalement le sulfolane et une phase d'extrait au contresolvant riche en isoprène et on soumet la phase d'extrait au contre-solvant riche en isoprène à une distillation fractionnée de manière à en éliminer le contre-solvant;

(3) un stade d'élimination des hydrocarbures lourds pour éliminer, par distillation fractionnée, les hydrocarbures, présents dans la fraction d'hydrocarbures, qui bouillent à une température au-dessus du point d'ébullition de l'isoprène.

2. Un procédé selon la revendication 1, caractérisé en ce que l'ordre des stades est le stade d'élimination des hydrocarbures légers, suivi du stade d'extraction liquide-liquide en deux étapes, suivi du stade d'élimination des hydrocarbures lourds.

3. Un procédé selon la revendication 1 ou la revendication 2, caractérisé par:

(1) une distillation fractionnée de la fraction d'hydrocarbures de façon qu'on obtienne une première fraction de distillat, comprenant principalement des hydrocarbures qui bouillent à une température au-dessous du point d'ébullition de l'isoprène, et une première fraction résiduelle comprenant le reste de la fraction d'hydrocarbures;

(2) l'extraction de l'isoprène et des hydrocarbures qui bouillent au-dessus du point d'ébullition de l'isoprène de la première fraction résiduelle par une série d'étapes comprenant:

(a) l'exposition de la première fraction résiduelle à une extraction liquide-liquide par mise en contact de la fraction résiduelle avec un sulfolane de façon qu'on obtienne une première phase de raffinat riche en pentane normal et en pentènes et une première phase d'extrait comprenant le sulfolane et le reste du résidu;

(b) l'exposition de la première phase d'extrait à une extraction liquide-liquide par le contre-solvant comprenant un alcane en $C_6$ ou supérieur de façon qu'on obtienne une seconde phase de raffinat comprenant principalement le sulfolane et une seconde phase d'extrait comprenant principalement le contre-solvant, l'isoprène et les autres hydrocarbures bouillant à une température au-dessus du point d'ébullition de l'isoprène, et

(c) une distillation fractionnée de la seconde phase d'extrait de façon qu'on obtienne une seconde phase résiduelle comprenant principalement le contre-solvant et une seconde phase de distillat comprenant principalement de l'isoprène et des hydrocarbures bouillant à une température au-dessus du point d'ébullition de l'isoprène, et

(3) une distillation fractionnée du second distillat de façon qu'on obtienne une troisième fraction résiduelle comprenant principalement des hydrocarbures bouillant audessus du point d'ébullition de l'isoprène et une troisième fraction de distillat comprenant principalement de l'isoprène.

4. Un procédé selon l'une quelconque des revendications précédentes, caractérisé en ce que tout pentane normal restant présent dans la première phase d'extrait en est éliminé par strippage avant que la phase soit soumise à l'extraction liquide-liquide par le contre-solvant.

5. Un procédé selon l'une quelconque des revendications précédentes, caractérisé en ce que le sulfolane utilisé dans la première étape du stade d'extraction liquide-liquide est le sulfolane lui-même.

6. Un procédé selon l'une quelconque des revendications précédentes, caractérisé en ce que le contre-solvant utilisé dans la seconde étape de l'extraction liquide-liquide est un alcane de $C_6$ à $C_8$.

**Patentansprüche**

1. Verfahren zur Gewinnung von Isopren aus einer $C_5$-Kohlenwasserstoff-Fraktion, die aus einer Mischung von Pentanen, z.B. Isopentan und normal-Pentan, Pentenen, Pentadienen einschliesslich Isopren und Pentinen besteht, und die möglicherweise auch geringe Mengen verschiedener Kohlenwasserstoffe mit C-Zahlen kleiner und/oder grösser $C_5$ enthält, dadurch gekennzeichnet, dass man die Kohlenwasserstoff-Fraktion den folgenden Stufen beliebigmit der Ausnahme, dass die Stufe zur Entfernung der schweren Kohlenwasserstoffe nicht der Stufe für eine Flüssig-Flüssig-Extraktion vorausgehen soll—aussetzt:

(i) Stufe zur Entfernung der leichten Kohlenwasserstoffe mittels fraktionierter Destillation, um solche in der Kohlenwasserstoff-Fraktion enthaltene Kohlenwasserstoffe zu entfernen, die bei einer Temperatur sieden,

die unterhalb des Siedepunkts des Isoprens liegt;

(ii) Stufe der Flüssig-Flüssig-Extraktion unter Verwendung eines Sulfolans als Extrahiermittel im ersten Schritt, zur Bildung einer an normal-Pentan und Pentanen reichen ersten Raffinatphase und einer an Isopren reichen Sulfolan-Extraktphase, wobei die Sulfolan-Extraktphase unter Einsatz eines Gegen-Lösungsmittels einem zweiten Schritt der Flüssig-Flüssig-Extraktion unterworfen wird und das Gegen-Lösungsmittel ein $C_6$- oder höheres Alkan als Extrahiermittel Anwendung findet zur Bildung einer hauptsächlich Sulfolan enthaltenden zweiten Raffinatphase und einer isoprenreichen Gegen-Lösungsmittel-Extraktphase und dass man zur Entfernung des Gegen-Lösungsmittels die Gegenlösungmittel-Extraktphase fraktioniert destilliert;

(iii) Stufe der Entfernung der schweren Kohlenwasserstoffe mittels fraktionierter Destillation, um solche in der Kohlenwasserstofffraktion enthaltene Kohlenwasserstoffe zu entfernen, die bei einer Temperatur sieden, die bei einer oberhalb des Siedepunkts des Isoprens liegenden Temperatur sieden.

2. Verfahren nach Anspruch 1, dadurch gekennzeichnet, dass der Reihe nach die Stufen-Entfernung der leichten Kohlenwasserstoffe — Zwei-Schritt-Flüssig-Flüssig-Extraktionsstufe—Entfernung der schweren Kohlenwasserstoffe—durchlaufen werden.

3. Verfahren nach Anspruch 1 oder 2, dadurch gekennzeichnet, dass man

(i) die Kohlenwasserstoff-Fraktion fraktioniert destilliert, um eine erste Destillat-Fraktion, die hauptsächlich Kohlenwasserstoffe, die bei einer Temperatur oberhalb des Siedepunkts des Isoprens sieden und eine erste Rückstands-Fraktion, die den Rest der Kohlenwasserstoff-Fraktion enthält, zu erhalten;

(ii) Isopren und Kohlenwasserstoffe, die über dem Siedepunkt des Isoprens sieden, aus der ersten Rückstandsfraktion auf der folgenden Schrittserie extrahiert:

(a) Flüssig-Flüssig-Extraktion der ersten Rückstandsfraktion durch Zusammenbringen der Rückstandsfraktion mit einem Sulfolan, um eine erste an normal-Pentan und Pentenen reichen Raffinatphase und eine erste Extraktionsphase mit Sulfolanen und dem Rest des Rückstands zu erhalten;

(b) Flüssig-Flüssig-Extraktion der ersten Extraktphase mit dem aus $C_6$- oder höheren Alkanen bestehenden Gegenlösungsmittel zur Gewinnung einer hauptsächlich aus Sulfolan bestehenden zweiten Raffinatphase und einer zweiten Extraktphase, die hauptsächlich das Gegenlösungsmittel, Isopren und andere Kohlenwasserstoffe, die bei einer oberhalb des Siedepunkts des Isoprens liegenden Temperatur sieden, enthält;

(c) Fraktionierte Destillation der zweiten Extrakphase zur Gewinnung einer hauptsächlich das Gegenlösungsmittel enthaltenden zweiten Rückstandsfraktion und einer zweiten Destillatfraktion, die im wesentlichen aus Isopren und Kohlenwasserstoffen, die bei einer oberhalb des Siedepunkts des Isoprens liegenden Temperatur sieden, besteht; und

(iii) dass man das zweite Destillat zur Gewinnung einer dritten Rückstandsfraktion, die hauptsächlich Kohlenwasserstoffe enthält, die bei einer oberhalb des Siedepunkts des Isoprens liegenden Temperatur sieden, und einer dritten Destillatfraktion, die im wesentlichen aus Isopren besteht, fraktioniert destilliert.

4. Verfahren nach einem der vorhergehenden Ansprüche, dadurch gekennzeichnet, dass jedes verbleibende normal-Pentan, das in der ersten Extraktphase vorhanden ist, daraus durch Abziehen entfernt wird, bevor die Phase der Flüssig-Flüssig-Extraktion mit dem Gegenlösungsmittel unterworfen wird.

5. Verfahren nach einem der vorhergehenden Ansprüche, dadurch gekennzeichnet, dass man im ersten Schritt der Flüssig-Flüssig-Extraktion ein Sulfolan verwendet, das Sulfolan selbst ist.

6. Verfahren nach einem der vorhergehenden Ansprüche, dadurch gekennzeichnet, dass das im zweiten Schritt der Flüssig-Flüssig-Extraktion verwendete Gegenlösungsmittel ein $C_6$- bis $C_8$-Alkan ist.

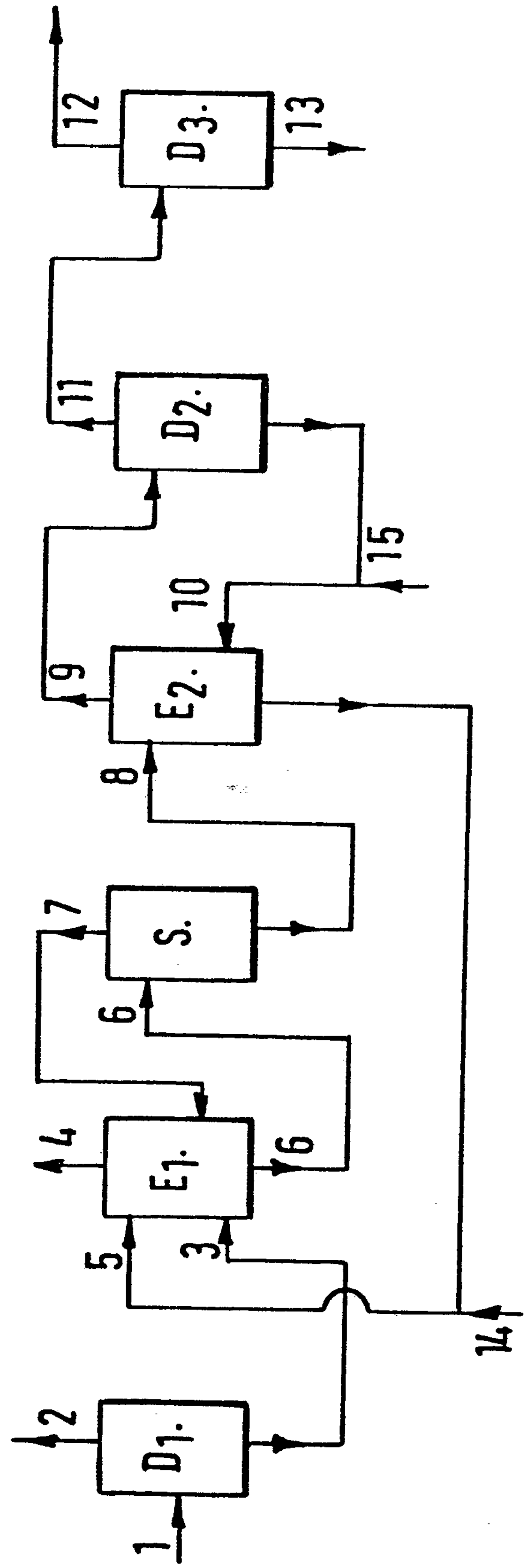

1
2
D1.
3
4
5
6
E1.
6
7
S.
8
9
10
E2.
11
D2.
12
13
D3.
14
15